# EUROPEAN PATENT SPECIFICATION

(11) **EP 1 904 136 B1**
(45) Date of publication and mention of the grant of the patent: **14.12.2011**
(21) Application number: 05768609.9
(22) Date of filing: 23.06.2005
(51) Int. Cl.: A61M 16/12

(54) **Breathing or Respiration Assistance Device**
Gerät zur Unterstützung der Atmung
Dispositif d'assistance respiratoire

(43) Date of publication of application: 02.04.2008
(73) Proprietor: ResMed Paris, 77550 Moissy Cramayel (FR)
(72) Inventor: CHALVIGNAC, Philippe, Hunters Hill, NSW 2110 (AU)
(74) Representative: Tetaz, Franck Claude Edouard
(86) International application number: PCT/IB2005/002326
(87) International publication number: WO 2006/136878

(56) References cited:
- EP-A- 0 878 207
- EP-A- 1 364 673
- WO-A-96/11718
- US-A- 5 701 883
- US-A1- 2005 115 564

## Description

### FIELD OF THE INVENTION

The present invention concerns a breathing assistance device for a patient.

The invention concerns a breathing assistance device also including the following features:
- a first gas inlet for receiving a primary respiratory gas and a first regulating means for regulating said primary respiratory gas,
- a second gas inlet for receiving a secondary respiratory gas and a second regulating means for regulating said secondary respiratory gas,
- a controlling means for controlling said first and second regulating means in order to mix said primary and secondary respiratory gases in controlled respective proportions into a controlled mix of gases,
- a feeding means for feeding said patient with said controlled mix of gases.

### TECHNICAL BACKGROUND

Breathing assistance devices such as mentioned above already exist.

Such devices comprise a central unit which includes a pressurised primary gas source such as a turbine for compressing the air from the ambient atmosphere (air being thus the primary respiratory gas). The first regulating means of the breathing assistance device can correspond to the turbine itself, with its associated control means (e.g. control of the speed of rotation of the turbine).

In other devices of the art, the first regulating means comprises the turbine and additional means (such as a proportional valve).

These devices also comprise a feeding means in the form of a respiratory duct for bringing the pressurised gas to the patient.

It is furthermore possible that the device additionally comprises a secondary respiratory duct between the central unit and the patient, said second duct being dedicated to evacuating the gases expired by the patient, such as carbon dioxide.

In such devices, the inlet of the turbine corresponds to the first gas inlet for the primary respiratory gas to be provided to the patient. Alternatively, the first gas inlet can be replaced by means allowing a connection to an external pressurised primary gas source (such as a fixed source of pressurised air e.g. in a hospital).

It is further known to incorporate into such breathing assistance devices a second gas inlet for receiving a secondary respiratory gas to be provided to said patient.

The secondary respiratory gas is typically oxygen. This secondary respiratory gas is mixed to air in a controlled proportion. Therefore the breathing assistance device is provided with a second regulating means controlled by a controlling means. This allows to feed the patient with pressurised gas comprising air having been enriched with oxygen in a controlled manner.

The secondary respiratory gas is generally provided by an external secondary gas source, such as a pressurised oxygen bottle or, e.g. in hospitals, a wall outlet for providing pressurised oxygen. The second gas inlet of the known devices therefore includes a connection means for connecting the external secondary gas source to the breathing assistance device. This connection means is fixed on a wall of the central unit.

In practice, the connection means for connecting the external secondary gas source to the breathing assistance device has in itself a significant volume.

Indeed, the secondary gas is typically pressurised oxygen which is provided to the breathing assistance device at a pressure which is between 4 and 6 atmospheres. (1 atmosphere = 101000 Pa)

And for safety reasons, the connection means to such a secondary gas source has to be robust enough to address the risk of leakage. The resulting connection means is thus of a significant size.

For oxygen as a secondary respiratory gas, there are standard connection means which make it possible to connect the respiratory unit to a pressurised secondary gas source wherever it is to be found. The existing devices thus use such standard connection means located on a wall of the central unit.

Finally, it is specified that the devices which include a second gas inlet for receiving a secondary respiratory gas such as oxygen are in practice used either for feeding the patient with a mix of primary and secondary respiratory gases, or with primary respiratory gas only. In other words, in many cases enriching the primary respiratory gas with the secondary respiratory gas is an option which is not permanently used.

To this end, a mixing means (such as a three way valve) is generally provided within the central unit to mix, only when it is desired, said primary and secondary respiratory gases.

Documents US 5,701,883, WO 96111718 and US 20051115564 give examples of such devices of the prior art that enable a mix of several gases.

The above being exposed, it is furthermore to be specified that a current trend of design for breathing assistance devices is to try to make them as small as possible.

It is indeed desired to obtain a device as small as possible, this increasing in particular the ability for the patient to move freely while being connected to the breathing assistance device.

However, if it is desired to be able to connect a secondary respiratory gas, the large size of the connecting means mentioned above is a limitation to the miniaturization of the breathing assistance device, and more precisely to its central unit.

The second regulating means constitutes a further limitation to miniaturization.

It is thus an object of the invention to overcome such limitation.

### SUMMARY OF THE INVENTION

In order to attain the above-mentioned object, the invention proposes a breathing assistance device as defined in claim 1.

In particular, the invention concerns a breathing assistance device for a patient comprising:
- a first gas inlet for receiving a primary respiratory gas and a first regulating means for regulating said primary respiratory gas, said first gas inlet and first regulating means being both located in a central unit of the breathing assistance device;
- a second gas inlet for receiving a secondary respiratory gas and a second regulating means for regulating said secondary respiratory gas;
- a controlling means for controlling said first and second regulating means in order to mix said primary and secondary respiratory gases in controlled respective proportions into a controlled mix of gases;
- a feeding means for feeding said patient with said controlled mix of gases, said feeding means being located between the central unit and the patient;
characterised in that said second gas inlet and second regulating means are both located in a secondary unit of the breathing assistance device, said secondary unit being removably connected to the central unit.

Preferable but not limited aspects of such a breathing assistance device are the following:
- the secondary unit is removably connected to the central unit via a male and female joint or by a connection duct for the secondary respiratory gas, the connection duct having a diameter from 4 to 8 mm;
- the second gas inlet comprises a connection means being adapted to be connected to an external pressurised gas source;
- the second regulating means comprises a pressure reducing means and/or a proportional means that may be electrically operated;
- the controlling means is located within the central unit;
- the second regulating means is controlled by the controlling means;
- the secondary unit comprises a data link allowing a transmission of data between the second regulating means and the controlling means of the central unit;
- the secondary unit comprises a power supply link for transmitting power from the central unit to the second regulating means;
- the secondary unit comprises a controlling electronic board being interposed between the regulating means and the data and power supply links, and being adapted to process data for controlling the second regulating means;
- the secondary unit comprises at least a gas flow sensor and a pressure sensor, both being connected to the controlling electronic board;
- the secondary unit comprises a central groove for storing said connection duct, so that the secondary unit may have a diabolo shape.

According to a further aspect of the invention, there is proposed a secondary unit for a breathing assistance device, the breathing assistance device being capable of feeding a patient with a mix of at least two respiratory gases and comprising a central unit, characterised in that the secondary unit is removably connected to the central unit, and is adapted to feed the central unit with a regulated flow of a respiratory gas coming from an external pressurised gas source.

Preferable but not limited aspects of such a secondary unit are the following:
- the secondary unit is removably connected to the central unit via a male and female joint or by a connection duct, the connection duct having a diameter from 4 to 8 mm;
- the secondary unit comprises a single inlet having a connection means adapted to be connected to an external pressurised gas source;
- the secondary unit comprises a power supply link for transmitting power from the central unit to secondary unit;
- the secondary unit comprises a regulating means for regulating said respiratory gas, the regulating means comprising a pressure reducing means and/or a proportional means;
- the secondary unit comprises a controlling electronic card for controlling the regulating means, the data and power supply links being connected to the controlling electronic card;
- the secondary unit comprises a central groove for storing the connection duct, so that the secondary unit may have a diabolo shape.

According to another aspect of the invention, there is provided a central unit for a breathing assistance device, the breathing assistance device being capable of feeding a patient with a mix of gases comprising a primary respiratory gas and a secondary respiratory gas, characterised in that the central unit comprises a gas inlet for receiving a regulated flow of the secondary respiratory gas, and a mixing means for selectively mixing said regulated flow of the secondary gas with said primary gas in order to obtain the desired mix of gases.

Preferable but not limited aspects of such a central unit are the following:
- the mixing means is a three way valve;
- the central unit comprises a controlling means for controlling a regulating means located outside said central unit, said regulating means regulating the flow of the secondary respiratory gas that reaches the inlet of the central unit;
- the inlet is adapted for receiving ducts having diameters from 4 to 8 mm.

### BRIEF DESCRIPTION OF THE DRAWINGS

Other characteristics and advantages of the invention will become clear from the following description which is only given for illustrative purposes and is in no way limitative and should be read with reference to the attached drawings on which:
- Figure 1 is a schematic representation of a breathing assistance device according to the invention;
- Figure 2 is a schematic representation of a breathing assistance device according to another embodiment of the invention;
- Figure 3 is a schematic representation of a secondary unit of a breathing assistance device according to the invention;
- Figure 4 is a schematic sectional view of an embodiment of a secondary unit of the breathing assistance device according to the invention.

### DETAILED DESCRIPTION OF THE DRAWINGS

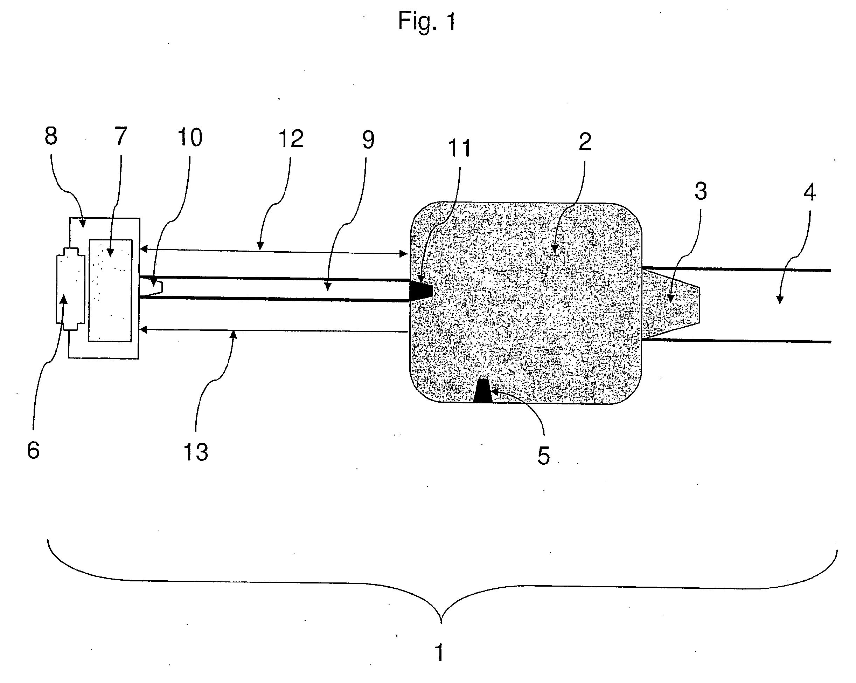

We shall first describe the breathing assistance device of the invention with reference to figure 1.

The breathing assistance device 1 of the invention comprises a central unit 2 that is capable of providing a respiratory gas to a patient.

The central unit 2 is provided with an outlet 3 from which the respiratory gas is brought from the central unit 2 to the patient via a feeding means 4. Such an outlet 3 may be for instance a rubber element having a tapered shape.

The feeding means 4 is connected to the outlet 3 of the central unit 2 and allows the patient to be fed with the respiratory gas coming from the central unit 2 of the breathing assistance device 1.

This feeding means 4 may constitute the single respiratory duct between the patient and the central unit 2.

The proximal end of this duct is generally connected to the patient with a device adapted for tracheotomy or a mask.

Alternatively, the feeding means 4 may be associated to a second duct for the patient to reject expiratory gases.

The breathing assistance device 1 of the invention is foreseen for providing the patient with a respiratory gas being composed of a mix of a primary respiratory gas and a secondary respiratory gas, both gases being provided in controlled proportions.

Therefore the central unit 2 of the breathing assistance device 1 of the invention comprises a first gas inlet 5 for receiving a primary respiratory gas and a first regulating means (not represented) for regulating said primary respiratory gas.

The first gas inlet 5 is arranged for collecting ambient air, the ambient air being the primary respiratory gas to be provided to the patient.

Alternatively, the first gas inlet can be replaced by means allowing a connection to an external pressurised primary gas source (such as a fixed source of pressurised air, e.g. in a hospital).

The first regulating means is generally a turbine, preferably of a small size, which provides a controlled flow of the primary respiratory gas. This turbine may optionally be associated to another regulating device, such as a proportional valve, in order to increase the control of the flow of the primary respiratory gas.

The breathing assistance device 1 of the invention is also capable of providing the patient with a secondary respiratory gas.

To this end, the breathing assistance device 1 of the invention comprises a second gas inlet 6 and a second regulating means 7 for respectively receiving and controlling a secondary respiratory gas.

As exposed above, the second gas inlet 6 has a significant size (due in particular to security standards).

However, according to the invention, the second gas inlet 6 and the second regulating means 7 are not located within the central unit 2. They are namely located in a secondary unit 8 which is distinct from the central unit 2.

A mixing means, such as a three way valve, may be provided within the central unit 2 to mix said primary and secondary respiratory gases.

Using such a secondary unit 8 transforms the breathing assistance device in a modular breathing assistance device.

The secondary unit 8 is namely removably connected to the central unit 2. Therefore when it is desired to feed the patient with a mix of respiratory gases, that is comprising a secondary respiratory gas, the secondary unit 8 (connected to an external respiratory gas source) is coupled with the central unit 2.

When the patient needs to be fed only with the primary respiratory gas, the secondary unit 8 is not used. In this case, the breathing assistance device 1 consists only in the central unit 2.

When the secondary unit 8 and the central unit 2 are coupled, a connection duct 9 may thus be provided between the secondary unit 8 and the central unit 2.

Such a connection duct 9 allows a transmission of the secondary respiratory gas from the secondary unit 8 to the central unit 2. The connection duct 9 is namely connected to a gas outlet 10 (such as a rubber element having a tapered shape) of the secondary unit 8 at one end and to a gas inlet 11 of the central unit 2 at the other end.

As the secondary respiratory gas is regulated by the second regulating means 7 within the secondary unit 8, e.g. regarding its pressure, the security standards that the connection duct 9 has to fulfil are reduced. Therefore, the connection duct 9 may have dimensions significantly less critical than the dimensions of standard ducts (that usually connect the external respiratory gas source to the breathing assistance device). Indeed, the connection duct 9 may have a diameter between 4 and 8 mm (that is relatively smaller than the diameter of standard ducts comprised between 12 and 16 mm) and is relatively flexible, thin and light.

Moreover the gas inlet 11 of the central unit 2 is adapted to be connected with the connection duct 9 and is therefore of small dimensions. The gas inlet 11 of the central unit 2 is namely particularly small relative to the second gas inlet 6 of the secondary unit 8.

Having such a small gas inlet 11 in the central unit 2 is a great asset, especially when the breathing assistance device 1 is to be used only for a primary respiratory gas. Indeed in this case, as exposed above, the breathing assistance device 1 consists only in the central unit 2 and is consequently small and light.

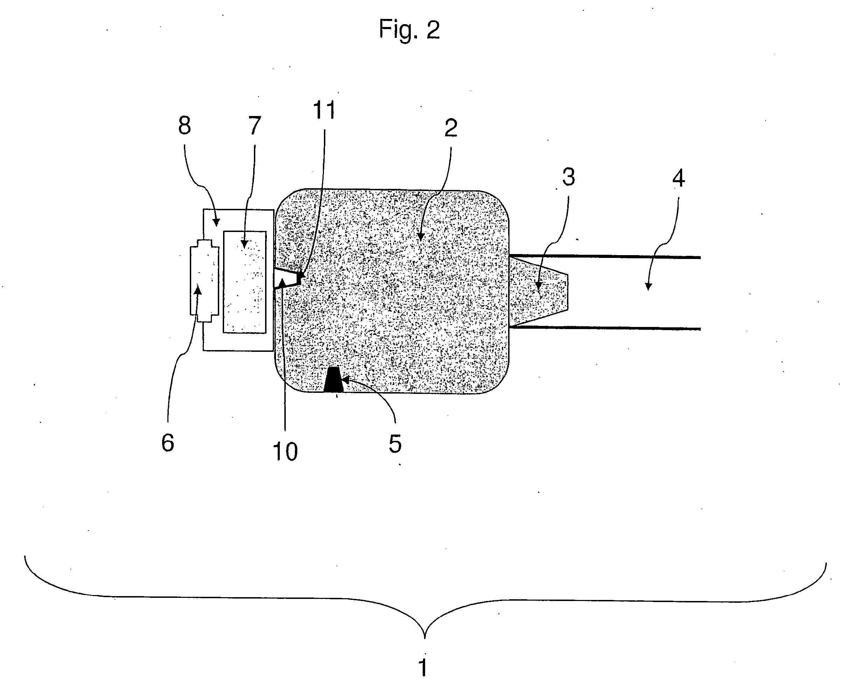

According to another embodiment of the invention as illustrated in figure 2, the secondary unit 8 is manufactured such that it may be directly coupled to the central unit 2, that is without any connection duct.

The secondary unit 8 may for example have a shape to be directly plugged on one side of the central unit 2. In this example, the central unit 2 and the secondary unit 8 may be coupled via a male and female joint.

In this case, the gas outlet 10 of the secondary unit 8 is adapted to be directly connected with the gas inlet 11 of the central unit 2. Once again, both gas outlet 10 and gas inlet 11 may be of small dimensions. Indeed, the secondary respiratory gas having been regulated within the secondary unit 8, e.g. regarding its pressure, the security standards that the gas outlet 10, and consequently the gas inlet 11, have to fulfil are reduced.

The breathing assistance device 1 of the invention further comprises a data link 12 provided between the secondary unit 8 and the central unit 2.

Indeed, the central unit 2 comprises a controlling means (not represented) for controlling, among others, the second regulating means 7 of the secondary unit 8.

This control is made possible thanks to the data link 12 which connects the controlling means of the central unit 2 and the second regulating means 7.

In a preferred embodiment of the invention, a power supply link 13 may be provided between the secondary unit 8 and the central unit 2.

Indeed, the regulating means 7 may be electrically operated and will in this case need a power supply. The central unit 2 being generally provided with a power supply, such as an internal battery or an external power supply, the power supply link 13 connected to the power supply of the central unit 2 feeds the second regulating means7 with power when needed.

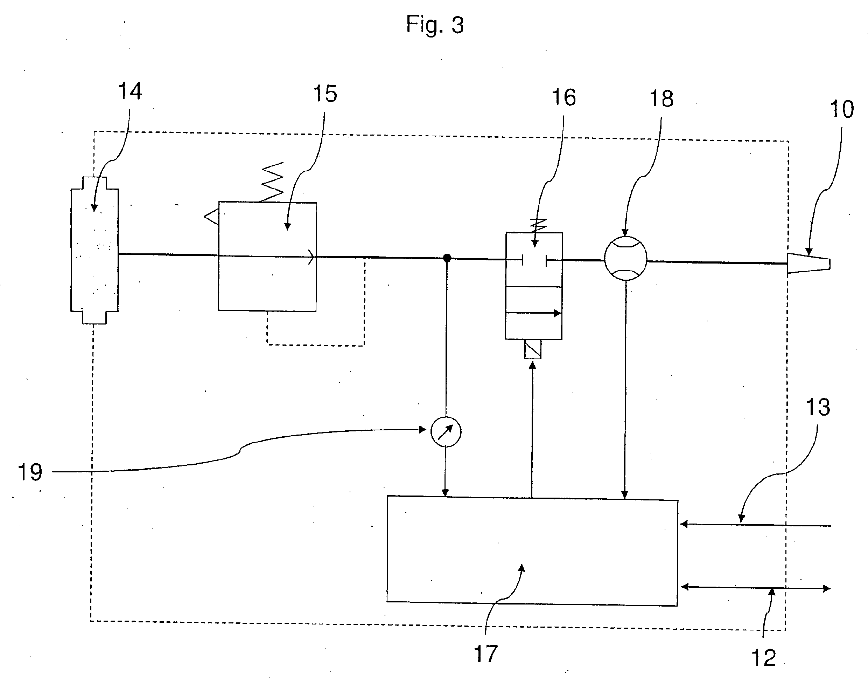

Figure 3 is a schematic representation of a secondary unit 8 of the breathing assistance device 1 according to the invention.

This secondary unit 8 comprises a connection means 14 which, corresponds to the second gas inlet 6.

The connection means 14 is adapted to be connected to an external pressurised source which provides a secondary respiratory gas.

As stated above, the secondary respiratory gas is typically pressurised oxygen which is provided to the breathing assistance device 1 at a pressure between 4 and 6 atmospheres.

Therefore, the connection means 14 has to be robust enough to address a risk of leakage, and is thus of a significant size.

As a consequence, the breathing assistance device 1 of the invention is preferably provided with a secondary unit 8 having a standard connection means 14, which make it possible to connect the breathing assistance device 1 to a pressurised secondary gas source wherever it is to be found.

As described before, the secondary unit 8 comprises a second gas regulating means 7.

The object of this second gas regulating means 7 is to control the secondary respiratory gas to be provided from the secondary unit 8 to the central unit 2 through the connection duct 9.

Indeed, the external pressurised gas source generally provides a secondary respiratory gas at high pressures. As the secondary respiratory gas is to be provided to the patient, this pressure has to be decreased. Therefore the second regulating means 7 comprises a pressure reducing means 15, such as a reducing valve.

The second regulating means 7 may further comprise a proportional means 16, such as a proportional valve. This proportional means 16 is capable of controlling the flow of the secondary respiratory gas to be provided to the patient.

Preferably, the inlet of the pressure reducing means 15 is coupled with the second gas inlet 6, the outlet of the pressure reducing means 15 is coupled with the inlet of the proportional means 16, and the outlet of the proportional means is coupled with the gas outlet 10 of the secondary unit 8.

In a preferred embodiment of the invention, the proportional means 16 is electrically operated. The proportional means may therefore be a proportional electrovalve. In such a case, the proportional means 16 is powered via the power supply link 13.

The operation of the second regulating means 7, and in particular of the proportional valve 16, is made possible thanks to the data transmitted from the controlling means of the central unit 2 through the data link 12.

It is therefore possible to set the characteristics that the flow of the secondary respiratory gas has to fulfil, e.g. the pressure of oxygen, directly on the central unit 2. A data processing means is namely provided within the central unit 2 in order to command the controlling means, which then controls the second regulating means 7 of the secondary unit 8.

Such data from the controlling means may directly control the second regulating means 7, being thus directly connected to the proportional means 16 for example.

Alternatively, a controlling electronic board 17 may be provided within the secondary unit 8 for processing the data coming from the controlling means of the central unit 2, and controlling the second regulating means 7 in consequence.

The power supply link 13 is preferably connected to the controlling electronic board 17 which transmits to the second regulating means 7 the power needed.

Measurement means may further be provided within the secondary unit 8 in order to increase the control of the second regulating means 7.

Thus, there is preferably provided a gas flow sensor 18, such as a hot wire sensor, and a pressure sensor 19.

In the case the second regulating means 7 comprises both a pressure reducing means 15 and a proportional means 16, the pressure sensor 19 is arranged to measure the pressure of the secondary respiratory gas between the pressure reducing means 15 and the proportional means 16. The gas flow sensor 18 is in this case arranged to measure the flow of the secondary respiratory gas coming from the proportional means 16.

The measurement means of the secondary unit 8 are either directly connected to the controlling means of the central unit 2, or connected to this controlling means via the controlling electronic board 17 of the secondary unit 8.

Using a controlling electronic board 17 is therefore a great advantage for the miniaturisation of the device. Indeed, it allows to reduce the number of data wires between the secondary unit 8 and the central unit 2, a single data wire being necessary as the data are directly processed within the controlling electronic board 17.

Thus, the coupling of the central unit 2 and the secondary unit 8 is simple as only three links are required, that is a link for the secondary respiratory gas (directly by connecting the gas outlet 10 and the gas inlet 11 together or via a connection duct 9), a single data wire 12 and a single power supply wire 13.

Moreover, both data and power supply wires may be gathered into a single and relatively small transmission cable.

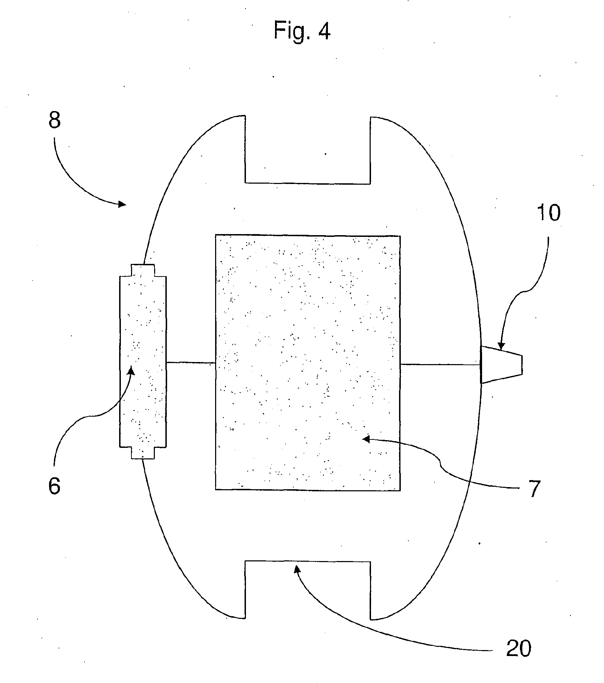

Finally, figure 4 is a schematic sectional view of an embodiment of the secondary unit 8 of the breathing assistance device according to the invention. Such a secondary unit 8 is to be coupled to the central unit 2 via a connection duct 9.

In this embodiment, a central groove 20 extending all around the secondary unit 8 is provided. Such a central groove 20 has dimensions so that the connection duct 9 may be stored therein. More precisely, the connection duct 9 is arranged within the groove 20 in order to be wrapped around of the secondary unit 8.

In the same manner, the data and power supply wires may be stored within the central groove 20.

Therefore, when a secondary respiratory gas has not to be provided to the patient, the secondary unit 8 may be removed from the central unit 2. to this end, the connection duct 9 and the data and power supply links (12;13) are disconnected from the central unit 2.

Such a disconnected secondary unit 8 having to be stored, it is convenient to dispose the connection duct 9, and eventually the data and power supply links (12;13), around the secondary unit 8 within the central groove 20.

A secondary unit 8 comprising a central groove 20 has for example a diabolo shape, as illustrated in figure 4.

## Claims

1. Breathing assistance device for a patient, comprising;
- a central unit (2), the central unit comprising a first gas inlet (5) for receiving a primary respiratory gas, a first regulating means for regulating said primary respiratory gas, a secondary gas inlet (11) adapted for receiving a supply of a regulated secondary respiratory gas, and a controlling means;
- a secondary unit (8) distinct from the central unit (2) and comprising a second gas inlet (6, 14) for receiving a secondary respiratory gas, a second regulating means (7) with a pressure reducing means (15) for regulating said secondary respiratory gas, and a gas outlet (10) for the regulated secondary respiratory gas; and
- a feeding means (4) to be located between the central unit (2) and the patient;
wherein the gas outlet (10) of the secondary unit (8) is configured to removably couple to the secondary gas inlet (11) of the central unit (2), and wherein said controlling means is adapted to control said first and second regulating means in order to mix said primary and secondary respiratory gases in controlled respective proportions into a controlled mix of gases that is fed to the patient through the feeding means,
**characterised in that** the dimensions of the secondary gas inlet (11) are smaller than the dimensions of the second gas inlet (6), wherein the controlling means is made to control the pressure reducing means (15) to decrease the pressure of the secondary respiratory gas to be provided to the secondary gas inlet (11) of the central unit (2).

2. Breathing assistance device according to claim 1, **characterised In that** the gas outlet (10) of the secondary unit (8) is directly coupled to the secondary gas inlet (11) of the central unit (2) via a male and female joint.

3. Breathing assistance device according to claim 1, **characterised in that** the gas outlet (10) of the secondary unit (8) is coupled to the secondary gas inlet (11) of the central unit (2) by a connection duct (9) for the secondary respiratory gas.

4. Breathing assistance device according any one of claim 1 to 3, **characterised in that** the second gas inlet (6) comprises a connection means (14) being adapted to be connected to an external pressurised gas source.

5. Breathing assistance device according to any one of claims 1 to 4, **characterised in that** the second regulating means (7) comprises a proportional means (16).

6. Breathing assistance device according to claim 5, **characterised in that** the proportional means (16) is a proportional valve being electrically operated.

7. Breathing assistance device according to any one of claims 1 to 6, **characterised in that** the secondary unit (8) comprises a data link (12) allowing a transmission of data between the second regulating means (7) and the controlling means of the central unit (2).

8. Breathing assistance device according to claim 7, **characterised in that** the secondary unit (8) comprises a power supply link (13) for transmitting power from the central unit (2) to the second regulating means (7),

9. Breathing assistance device according to claim 8, **characterised in that** the secondary unit (8) comprises a controlling electronic board (17) being interposed between the regulating means (7) and the data and power supply links (12,13), and being adapted to process data for controlling the second regulating means (7).

10. Breathing assistance device according to claim 9, **characterised in that** the secondary unit (8) comprises at least a gas flow sensor (18) and a pressure sensor (19), both being connected to the controlling electronic board (17).

11. Breathing assistance device according any one of claims 1 to 10, **characterised in that** the secondary unit (8) comprises at least a gas flow sensor (18) and a pressure sensor (19), both being directly connected to the controlling means of the central unit (2).

12. Breathing assistance device according any one of claims 3 to 11, **characterised in that** the connection duct (9) between the secondary unit (8) and the central unit (2) has a diameter of 4 to 8 mm.

13. Breathing assistance device according any one of claims 3 to 12, **characterised in that** the secondary unit (8) comprises a central groove (20) for storing said connection duct (9).

14. Breathing assistance device according to claim 13, **characterised In that** the secondary unit (8) has a diabolo shape.

15. Breathing assistance device according to any one of claims 1 to 14, **characterised in that** the secondary gas inlet (11) is adapted for receiving ducts having diameters from 4 to 8 mm.

16. Breathing assistance device according to any one of claims 1 to 15, **characterised in that** the first regulating means is a turbine that provides a controllable flow of the primary respiratory gas.

17. Breathing assistance device according to any one of claims 1 to 16, **characterised in that** the central unit (2) comprises mixing means for mixing the primary and secondary respiratory gases within the central unit (2) before being provided to the feeding means (4).

18. Breathing assistance device according to claim 17, **characterised in that** the mixing means is a three way valve.

19. Breathing assistance device according to any one of claims 1 to 18, **characterised in that** the secondary unit has a shape adapted to be directly plugged on one side of the central unit (2).

20. Secondary unit as defined in any one of claims 1 to 19 adapted to be removably coupled to the central unit of the breathing assistance device of any one of claims 1 to 19.

21. Central unit as defined in any one of claims 1 to 19 adapted to be removably coupled to the secondary unit of the breathing assistance device of any one of claims 1 to 19.

## Patentansprüche

1. Atmungsunterstützungsgerät für einen Patienten, das Folgendes umfasst:
- eine Zentraleinheit (2), wobei die Zentraleinheit Folgendes umfasst: einen ersten Gaseinlass (5) zum Empfangen eines primären Atemgases; ein erstes Regulierungsmittel zum Regulieren des primären Atemgases; einen sekundären Gaseinlass (11), der dafür ausgelegt ist, eine Zufuhr eines regulierten sekundären Atemgases zu empfangen; und ein Steuerungsmittel;
- eine Sekundäreinheit (8), die von der Zentraleinheit (2) getrennt ist und Folgendes umfasst: einen zweiten Gaseinlass (6, 14) zum Empfangen eines sekundären Atemgases; ein zweites Regulierungsmittel (7) mit einem Druckminderungsmittel (15) zum Regulieren des sekundären Atemgases; und einen Gasauslass (10) für das regulierte sekundäre Atemgas; und
- ein Zuleitungsmittel (4), das zwischen der Zentraleinheit (2) und dem Patienten anzuordnen ist;
wobei der Gasauslass (10) der Sekundäreinheit (8) dafür konfiguriert ist, lösbar mit dem sekundären Gaseinlass (11) der Zentraleinheit (2) gekoppelt zu werden, und wobei das Steuerungsmittel dafür ausgelegt ist, das erste und das zweite Regulierungsmittel zu steuern, um das primäre und das sekundäre Atemgas in kontrollierten jeweiligen Anteilen zu einem kontrollierten Gasgemisch zu vermischen, das dem Patienten durch das Zuleitungsmittel zugeleitet wird,
**dadurch gekennzeichnet, dass** die Abmessungen des sekundären Gaseinlasses (11) kleiner sind als die Abmessungen des zweiten Gaseinlasses (6), wobei das Steuerungsmittel veranlasst wird, das Druckminderungsmittel (15) so anzusteuern, dass es den Druck des sekundären Atemgases, das dem sekundären Gaseinlass (11) der Zentraleinheit (2) zugeführt werden soll, mindert.

2. Atmungsunterstützungsgerät nach Anspruch 1, **dadurch gekennzeichnet, dass** der Gasauslass (10) der Sekundäreinheit (8) direkt mit dem sekundären Gaseinlass (11) der Zentraleinheit (2) über eine Steckverbindung gekoppelt ist.

3. Atmungsunterstützungsgerät nach Anspruch 1, **dadurch gekennzeichnet, dass** der Gasauslass (10) der Sekundäreinheit (8) mit dem sekundären Gaseinlass (11) der Zentraleinheit (2) mittels eines Verbindungskanals (9) für das sekundäre Atemgas gekoppelt ist.

4. Atmungsunterstützungsgerät nach einem der Ansprüche 1 bis 3, **dadurch gekennzeichnet, dass** der zweite Gaseinlass (6) ein Verbindungsmittel (14) umfasst, das dafür ausgelegt ist, mit einer externen Druckgasquelle verbunden zu werden.

5. Atmungsunterstützungsgerät nach einem der Ansprüche 1 bis 4, **dadurch gekennzeichnet, dass** das zweite Regulierungsmittel (7) ein Proportionalmittel (16) umfasst.

6. Atmungsunterstützungsgerät nach Anspruch 5, **dadurch gekennzeichnet, dass** das Proportionalmittel (16) ein elektrisch betätigtes Proportionalventil ist.

7. Atmungsunterstützungsgerät nach einem der Ansprüche 1 bis 6, **dadurch gekennzeichnet, dass** die Sekundäreinheit (8) eine Datenverbindung (12) umfasst, welche die Übertragung von Daten zwischen dem zweiten Regulierungsmittel (7) und dem Steuerungsmittel der Zentraleinheit (2) ermöglicht.

8. Atmungsunterstützungsgerät nach Anspruch 7, **dadurch gekennzeichnet, dass** die Sekundäreinheit (8) eine Stromversorgungsverbindung (13) zum Übertragen von Strom von der Zentraleinheit (2) zu dem zweiten Regulierungsmittel (7) umfasst.

9. Atmungsunterstützungsgerät nach Anspruch 8, **dadurch gekennzeichnet, dass** die Sekundäreinheit (8) eine Steuerungsplatine (17) umfasst, die zwischen dem Regulierungsmittel (7) und der Daten- und der Stromversorgungsverbindung (12, 13) angeordnet ist und dafür ausgelegt ist, Daten zum Steuern des zweiten Regulierungsmittels (7) zu verarbeiten.

10. Atmungsunterstützungsgerät nach Anspruch 9, **dadurch gekennzeichnet, dass** die Sekundäreinheit (8) mindestens einen Gasströmungssensor (18) und einen Drucksensor (19) umfasst, die beide mit der Steuerungsplatine (17) verbunden sind.

11. Atmungsunterstützungsgerät nach einem der Ansprüche 1 bis 10, **dadurch gekennzeichnet, dass** die Sekundäreinheit (8) mindestens einen Gasströmungssensor (18) und einen Drucksensor (19) umfasst, die beide direkt mit dem Steuerungsmittel der Zentraleinheit (2) verbunden sind.

12. Atmungsunterstützungsgerät nach einem der Ansprüche 3 bis 11, **dadurch gekennzeichnet, dass** der Verbindungskanal (9) zwischen der Sekundäreinheit (8) und der Zentraleinheit (2) einen Durchmesser von 4 bis 8 mm hat.

13. Atmungsunterstützungsgerät nach einem der Ansprüche 3 bis 12, **dadurch gekennzeichnet, dass** die Sekundäreinheit (8) eine mittige Nut (20) zum Aufnehmen des Verbindungskanals (9) umfasst.

14. Atmungsunterstützungsgerät nach Anspruch 13, **dadurch gekennzeichnet, dass** die Sekundäreinheit (8) eine Diaboloform hat.

15. Atmungsunterstützungsgerät nach einem der Ansprüche 1 bis 14, **dadurch gekennzeichnet, dass** der sekundäre Gaseinlass (11) dafür ausgelegt ist, Kanäle mit Durchmessern von 4 bis 8 mm aufzunehmen.

16. Atmungsunterstützungsgerät nach einem der Ansprüche 1 bis 15, **dadurch gekennzeichnet, dass** das erste Regulierungsmittel eine Turbine ist, die einen regelbaren Strom des primären Atemgases ausgibt.

17. Atmungsunterstützungsgerät nach einem der Ansprüche 1 bis 16, **dadurch gekennzeichnet, dass** die Zentraleinheit (2) ein Vermischungsmittel umfasst, um das primäre und das sekundäre Atemgas innerhalb der Zentraleinheit (2) zu vermischen, bevor es zu dem Zuleitungsmittel (4) geführt wird.

18. Atmungsunterstützungsgerät nach Anspruch 17, **dadurch gekennzeichnet, dass** das Vermischungsmittel ein Dreiwegventil ist.

19. Atmungsunterstützungsgerät nach einem der Ansprüche 1 bis 18, **dadurch gekennzeichnet, dass** die Sekundäreinheit eine Form hat, die dafür geeignet ist, direkt an eine Seite der Zentraleinheit (2) gesteckt zu werden.

20. Sekundäreinheit wie in einem der Ansprüche 1 bis 19 definiert, die dafür ausgelegt ist, lösbar mit der Zentraleinheit des Atmungsunterstützungsgerätes nach einem der Ansprüche 1 bis 19 gekoppelt zu werden.

21. Zentraleinheit wie in einem der Ansprüche 1 bis 19 definiert, die dafür ausgelegt ist, lösbar mit der Sekundäreinheit des Atmungsunterstützungsgerätes nach einem der Ansprüche 1 bis 19 gekoppelt zu werden.

## Revendications

1. Dispositif d'assistance respiratoire pour un patient comprenant :
une unité centrale (2), l'unité centrale comprenant une première entrée de gaz (5) pour recevoir un gaz respiratoire principal, des premiers moyens de régulation pour réguler ledit gaz respiratoire principal, une deuxième entrée de gaz (11) adaptée pour recevoir une alimentation d'un gaz respiratoire secondaire régulé, et des moyens de commande ;
une unité secondaire (8) distincte de l'unité centrale (2) et comprenant une deuxième entrée de gaz (6, 14) pour recevoir un gaz respiratoire secondaire, des deuxièmes moyens de régulation (7) avec des moyens de réduction de pression (15) pour réguler ledit gaz respiratoire secondaire, et une sortie de gaz (10) pour le gaz respiratoire secondaire régulé ; et
des moyens d'alimentation (4) destinés à être positionnés entre l'unité centrale (2) et le patient;
dans lequel la sortie de gaz (10) de l'unité secondaire (8) est configurée pour être couplée de manière amovible à l'entrée de gaz secondaire (11) de l'unité centrale (2), et dans lequel lesdits moyens de commande sont adaptés pour commander lesdits premier et deuxième moyens de régulation afin de mélanger lesdits gaz respiratoires principal et secondaire dans des proportions respectives contrôlées en un mélange de gaz contrôlé qui est fourni au patient par les moyens d'alimentation,
**caractérisé en ce que** les dimensions de l'entrée de gaz secondaire (11) sont plus petites que les dimensions de la deuxième entrée de gaz (6), dans lequel les moyens de commande sont prévus pour commander les moyens de réduction de pression (15) afin de diminuer la pression du gaz respiratoire secondaire destiné à être amené à l'entrée de gaz secondaire (11) de l'unité centrale (2).

2. Dispositif d'assistance respiratoire selon la revendication 1, **caractérisé en ce que** la sortie de gaz (10) de l'unité secondaire (8) est directement couplée à l'entrée de gaz secondaire (11) de l'unité centrale (2) via un joint mâle et femelle.

3. Dispositif d'assistance respiratoire selon la revendication 1, **caractérisé en ce que** la sortie de gaz (10) de l'unité secondaire (8) est couplée à l'entrée de gaz secondaire (11) de l'unité centrale (2) par un conduit de raccordement (9) pour le gaz respiratoire secondaire.

4. Dispositif d'assistance respiratoire selon l'une quelconque des revendications 1 à 3, **caractérisé en ce que** la deuxième entrée de gaz (6) comprend des moyens de raccordement (14) qui sont adaptés pour être raccordés à une source externe de gaz sous pression.

5. Dispositif d'assistance respiratoire selon l'une quelconque des revendications 1 à 4, **caractérisé en ce que** les deuxièmes moyens de régulation (7) comprennent des moyens proportionnels (16).

6. Dispositif d'assistance respiratoire selon la revendication 5, **caractérisé en ce que** les moyens proportionnels (16) sont une soupape proportionnelle qui est actionnée électriquement.

7. Dispositif d'assistance respiratoire selon l'une quelconque des revendications 1 à 6, **caractérisé en ce que** l'unité secondaire (8) comprend une liaison de données (12) permettant une transmission de données entre les deuxièmes moyens de régulation (7) et les moyens de commande de l'unité centrale (2).

8. Dispositif d'assistance respiratoire selon la revendication 7, **caractérisé en ce que** l'unité secondaire (8) comprend une liaison d'alimentation de puissance (13) pour transmettre la puissance de l'unité centrale (2) aux deuxièmes moyens de régulation (7).

9. Dispositif d'assistance respiratoire selon la revendication 8, **caractérisé en ce que** l'unité secondaire (8) comprend une carte électronique de commande (17) qui est intercalée entre les moyens de régulation (7) et les liaisons de données et d'alimentation de puissance (12, 13) et étant adaptée pour traiter des données afin de commander les deuxièmes moyens de régulation (7).

10. Dispositif d'assistance respiratoire selon la revendication 9, **caractérisé en ce que** l'unité secondaire (8) comprend au moins un capteur de flux de gaz (18) et un capteur de pression (19), les deux étant raccordés à la carte électronique de commande (17).

11. Dispositif d'assistance respiratoire selon l'une quelconque des revendications 1 à 10, **caractérisé en ce que** l'unité secondaire (8) comprend au moins un capteur de débit de gaz (18) et un capteur de pression (19), les deux étant directement raccordés aux moyens de commande de l'unité centrale (2).

12. Dispositif d'assistance respiratoire selon l'une quelconque des revendications 3 à 11, **caractérisé en ce que** le conduit de raccordement (9) entre l'unité secondaire (8) et l'unité centrale (2) a un diamètre de 4 à 8 mm.

13. Dispositif d'assistance respiratoire selon l'une quelconque des revendications 3 à 12, **caractérisé en ce que** l'unité secondaire (8) comprend une rainure centrale (20) pour stocker ledit conduit de raccordement (9).

14. Dispositif d'assistance respiratoire selon la revendication 13, **caractérisé en ce que** l'unité secondaire (8) a une forme de diabolo.

15. Dispositif d'assistance respiratoire selon l'une quelconque des revendications 1 à 14, **caractérisé en ce que** l'entrée de gaz secondaire (11) est adaptée pour recevoir des conduits ayant des diamètres de l'ordre de 4 à 8 mm.

16. Dispositif d'assistance respiratoire selon l'une quelconque des revendications 1 à 15, **caractérisé en ce que** les premiers moyens de régulation sont une turbine qui fournit un flux contrôlable du gaz respiratoire principal.

17. Dispositif d'assistance respiratoire selon l'une quelconque des revendications 1 à 16, **caractérisé en ce que** l'unité centrale (2) comprend des moyens de mélange pour mélanger les gaz respiratoires principal et secondaire à l'intérieur de l'unité centrale (2) avant qu'ils ne soient amenés aux moyens d'alimentation (4).

18. Dispositif d'assistance respiratoire selon la revendication 17, **caractérisé en ce que** les moyens de mélange sont une soupape à trois voies.

19. Dispositif d'assistance respiratoire selon l'une quelconque des revendications 1 à 18, **caractérisé en ce que** l'unité secondaire a une forme adaptée pour être directement branchée sur un côté de l'unité centrale (2).

20. Unité secondaire selon l'une quelconque des revendications 1 à 19, adaptée pour être couplée de manière amovible à l'unité centrale du dispositif d'assistance respiratoire selon l'une quelconque des revendications 1 à 19.

21. Unité centrale selon l'une quelconque des revendications 1 à 19, adaptée pour être couplée de manière amovible à l'unité secondaire du dispositif d'assistance respiratoire selon l'une quelconque des revendications 1 à 19.
